# EUROPEAN PATENT SPECIFICATION

(11) **EP 1 625 386 B1**
(45) Date of publication and mention of the grant of the patent: **23.06.2021**
(21) Application number: 04758045.1
(22) Date of filing: 24.03.2004
(51) Int. Cl.: G01N 21/77, G01N 21/86, G01N 33/543, A61B 5/00

(54) **ANALYTE CONCENTRATION DETECTION DEVICES AND METHODS**
VORRICHTUNGEN UND VERFAHREN ZUM NACHWEIS VON ANALYTKONZENTRATIONEN
DISPOSITIFS ET PROCÉDÉS DE DÉTECTION DE LA CONCENTRATION D'ANALYTES

(30) Priority: 24.03.2003 US 394230; 25.03.2003 US 456961 P; 28.03.2003 US 457996 P
(43) Date of publication of application: 15.02.2006
(60) Divisional application: 10180848.3
(73) Proprietor: Intuity Medical, Inc., Sunnyvale, CA 94085 (US)
(72) Inventor: HO, Wen, Plainsboro, NJ 08536 (US); ZANZUCCHI, Peter J., Princeton Junction, NJ 08550 (US); DESAI, Nitin V., Princeton Junction, NJ 08550 (US); MCBRIDE, Sterling E., Princeton, NJ 08540 (US); VARMA, Bobby, West Windsor, NJ 08550 (US); TSEKOUN, Alexei Guennadievich, Princeton, NJ 08540 (US)
(74) Representative: Cooley (UK) LLP
(86) International application number: PCT/US2004/008798
(87) International publication number: WO 2004/085995

(56) References cited:
- WO-A1-88/07666
- WO-A1-02/093144
- WO-A2-02/101359
- US-A- 4 637 403
- US-A- 4 815 843
- US-A- 5 110 724
- US-A- 5 114 350
- US-A- 5 208 163
- US-A- 5 670 031
- US-A- 5 701 181
- US-A- 5 801 057
- US-A- 5 986 754
- US-A1- 5 841 126
- US-B1- 6 312 888
- US-B1- 6 555 061

## Description

### FIELD OF THE INVENTION

The present invention is directed to techniques and devices for detection of the presence and/or concentration of an analyte and to techniques and devices for the effective collection, transportation and quantization of small quantities of body fluids.

### BACKGROUND OF THE INVENTION

A survey of the prior art reveals that numerous techniques and devices for performing an assay to determine the presence and/or concentrate of an analyte have been developed.

WO 88/07666 discloses a prior art device for conducting an assay to determine the concentration of an analyte in a sample of bodily fluid.

### SUMMARY OF THE INVENTION

According to the present invention, the state of the art has been advanced through the provision of devices and techniques such as those described further herein, for accurately, efficiently, and economically determining the presence and/or concentration of an analyte. According to the present invention, the state of the art has been advanced, especially, but not exclusively, within the context of personal glucose monitoring devices and techniques.

According to one aspect, the present invention provides a device for monitoring the concentration of an analyte present in bodily fluid, the device comprising a detector, the detector comprising a sensor, the sensor comprising a CMOS sensor, a CCD sensor, a photodiode or an infrared sensor, including both near-field and mid-field infrared sensors. Other sensing systems also contemplated within the scope of the present invention include ultraviolet and fluorescent sensing systems and electrochemical sensing systems, including reagent-less sensing approaches.

It is therefore to be understood that reference herein to the sensors or sensor array of the present invention may include any suitable sensor. The present invention is thus not limited to embodiments of the invention including CMOS or CCD sensors, photodiodes, infrared, fluorescent, ultraviolet or electrochemical sensors.

According to a further aspect, the present invention provides a device for conducting an assay to determine the concentration of an analyte in a sample of bodily fluid as set forth in claim 1.

It is to be understood that reference herein to first, second, third and fourth components does not limit the present invention to embodiments where each of these components is physically separable from one another. For example, a single physical element of the invention may perform the features of more than one of the claimed first, second, third or fourth components. Conversely, a plurality of separate physical elements working together may perform the claimed features of one of the claimed first, second, third or fourth components.

It is to be understood that "vertical" conveying of the sample as used herein refers to movement of the sample with respect to an assay pad, not with respect to an "up-down" direction per se.

### BRIEF DESCRIPTION OF THE DRAWINGS

Figure 1 is a schematic illustration of an example of an analyte detection arrangement useful in understanding the present invention.
Figure 2 is a schematic illustration of an example of another analyte detection arrangement useful in understanding the present invention.
Figure 3 is a schematic illustration of an analyte detection arrangement according to an aspect of the present invention.
Figure 4 is a schematic illustration of an analyte detection arrangement useful in understanding the present invention.
Figure 5 is a schematic illustration of an analyte detection arrangement useful in understanding the present invention.
Figure 6 is a schematic illustration of an analyte detection arrangement useful in understanding the present invention.
Figure 7 is a schematic illustration of an analyte detection arrangement
   useful in understanding the present invention.
Figure 8A is a schematic illustration of an assay arrangement and technique according to the present invention.
Figure 8B is a cross-section of Figure 8A taken along line 8B-8B.
Figure 9 is picture of an exemplary reaction spot according to the present invention.
Figure 10 is a scan along the dashed line of Figure 9.
Figure 11 is a schematic illustration of a layered test strip constructed according to the present invention.
Figure 12 is a schematic illustration of another layered test strip construction according to the present invention.
Figure 13 is a picture of a mesh wherein at least a portion thereof includes retained reagent.
Figure 14 is a schematic illustration of a further layered test strip construction according to the present invention.
Figure 15 is a schematic illustration of yet another layered test strip construction according to the present invention.
Figure 16 is a schematic illustration of a further layered test strip construction according to the present invention.
Figure 17 is a schematic illustration of an arrangement for the collection and analysis of a body fluid.
Figure 18 is a schematic illustration of the transportation of a body fluid within the context of the arrangement of Figure 17.
Figure 19 is a schematic of an arrangement for the transport and analysis of a body fluid, and illustrates an alternate construction.
Figure 20 is a schematic illustration of an arrangement for the transport and analysis of a body fluid, and illustrates a preferred construction.
Figure 21 is a schematic illustration of an arrangement for the transport and analysis of a body fluid.
Figure 22 is a schematic illustration of an arrangement for the transport and analysis of a body fluid.
Figure 23 is a schematic illustration of another alternative arrangement for the transport and analysis of a body fluid.
Figure 24 is a graphic representation comparing the signals generated by a detector array with and without a colorimetric reaction caused by the presence of an analyte.
Figure 25 is a graphic representation comparing the signals generated by a detector for various concentrations of glucose.
Figure 26 is a plot of detector signal versus glucose concentration according to a peak-averaging technique.
Figure 27 is a plot of the data of Figure 26 plotted with the logarithm of glucose concentration.
Figure 28 is a plot of detector signal versus glucose concentration according to a peak-value technique.
Figure 29 is plot of detector signal versus glucose concentration according to a peak-area technique.
Figure 30 is a plot of glucose concentration versus detector signal for four different test volumes showing the volume independence of the present invention.

### DETAILED DESCRIPTION OF THE INVENTION

Exemplary arrangements for the detection and measurement of the presence and/or concentration of an analyte, such as glucose, will now be described by reference to the drawing figures.

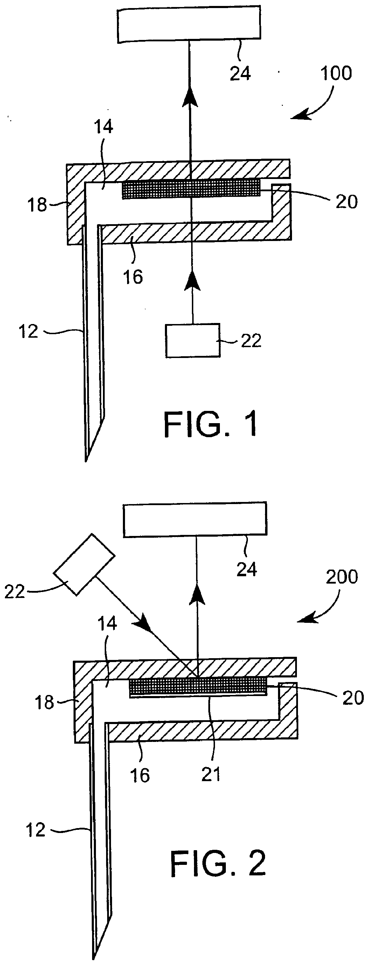

Figure 1 is one such arrangement 100 for the optical detection of the presence and/or concentration of an analyte such as glucose. The arrangement 100 can generally be described as a diffuse transmission arrangement.

According to this example the substance to be analyzed is transported via a conduit 12. When the substance to be analyzed comprises a sample of whole blood, interstitial fluid, or mixture thereof, the conduit 12 can be in the form of a hollow member or needle or any other skin piercing element, including, but not limited to lancets. The needle can be of a very narrow gage, or a so-called microneedle. Such a needle typically having a size on the order of 40-100 micrometers. It is to be understood, however, that diameters of less than 40 micrometers, or more than 100 micrometers are also contemplated within the scope of the present invention.

The microneedle may also act as a skin penetration member as well as a conduit. Alternatively, the skin penetration member may be in the form of a solid lancet (not shown), which acts to produce a sample of bodily fluid which is then transported via conduit, e.g.-12.

The substance to be analyzed is transported via the conduit 12 to a chamber 14. The chamber may have any suitable form. According to one example, the chamber 14 is formed by a lower member 16 and an upper member 18. These members 16, 18 can be constructed to allow light to be transmitted therethrough.

An assay pad 20 is provided within the chamber 14. The pad 20 receives the substance to be analyzed. The pad 20 is provided with a substance that reacts with the analyte. The results of this reaction is detectable and the data generated through detection is used to determine the presence and/or concentration of the analyte. The pad 20 is provided with a construction to achieve this objective, and others. A discussion of the specifics of the pad 20 will be deferred.

A light source 22 can be provided so as to produce light that is incident upon a first side of the pad 20. Any suitable light source is envisioned. According to a preferred embodiment, the light source comprises a light emitting diode (LED). The LED or light source 22 can provide incident light of any suitable wavelength. Wavelengths on the order of 500 - 700nm are suitable. For example, incident light with a wavelength on the order of 670nm may be transmitted from light source 22. At approximately 670nm, absorption of the incident light by hemoglobin tends to be reduced.

An optical detector 24 is positioned so as to receive light transmitted through a second side of the pad 20, the second side being opposite the first side. The optical detector can be a digital imaging device, such as a complementary metal oxide semiconductor (CMOS) device, a charge couple device (CCD), or any photodiode or any other suitable detector, including, but not limited to infrared sensors, including near and mid field infrared sensors. Electrochemical and reagent-less sensing techniques are also contemplated within the scope of the resent invention. The imaging device may comprise an array of individual detectors.

The implementation of digital imaging devices permits the analysis of samples that are not of uniform volume. This is accomplished at least in part by the ability of individual detectors to take readings, which are then averaged or combined to generate an overall reading. The larger the sample, the larger the "spot" formed on the pad 20, and the more individual detectors pick up a reading of the reaction occurring in the pad 20. Thus, unlike assays performed by conventional personal glucose monitoring devices and the like, the assay of the present invention is not dependent upon the volume of the sample being analyzed. When an assay is incorporated into a monitoring device wherein the monitoring is performed in an automated fashion, the user is often unable to determine if a sample of body fluid of a particular volume has been obtained. In conventional assays, the accuracy of the assay is directly linked to a specific volume of sample undergoing analysis (e.g. - "microtitration "). The present invention breaks this dependence, thereby providing robust and reliable devices, such as personal glucose monitoring devices, that can function effectively over a range of potential sample volumes. Other details of the detector 24, and associated components, will be deferred to later text.

The arrangement 100 may be incorporated into a larger overall device. According to one embodiment, the arrangement 100 is incorporated into a personal glucose-monitoring device. According to one alternative, the device is ambulatory. For example, the device is wearable by a user and autonomous in its operation. The arrangement 100 can be incorporated into devices, such as the ones described in U.S. Patent Publication No. US 2002/0087056 or US2003/0153900 (Serial No. 10/131,268).

In the following description of alternative arrangements, those elements that correspond to features already described in previous arrangement(s) have been designated with the same reference numbers.

An alternative arrangement 200 is depicted in Figure 2. The arrangement 200 can generally be described as a diffuse reflection arrangement.

While arrangement 200 is similar to the previously described arrangement 100, the light source 22 is provided such that it transmits light which is incident upon a first side of the assay pad 20. At least a portion of this incident light is reflected off of the first side. This reflected light can then be collected by the optical detector 24, which is disposed on the same side as the light source 22, relative to the test pad 20. Preferably, the light source 22 and the optical detector 24 are disposed, relative to the reaction chamber, on a side which is opposite from the side of the reaction chamber into which the conduit 12 introduces the substance to be analyzed. This orientation is shown in Figure 2.

In order to improve reflection of the incident light from the test pad, the test pad 20 may be provided with a blocking layer 21. Such a layer can be formed of any suitable material, such as zirconium oxide or titanium oxide.

Since only one side of the test pad is read by the detector, the reading taken by the detector 24 is insensitive to the contents of the chamber 14 since the contents of the chamber 14 are not in direct communication with the side of the test pad 20 being read.

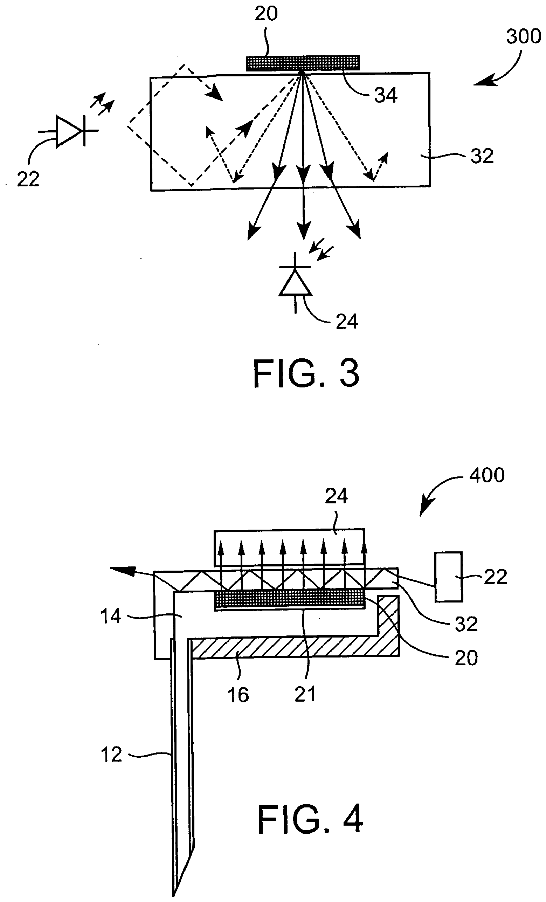

Another alternative arrangement is illustrated in Figure 3. The arrangement 300 can generally be described as an edge or waveguide illumination arrangement.

According to this example, a light source 22 is arranged such that a diverging light beam strikes an edge of a transparent waveguide member 32, and couples the transmitted light inside the waveguide, as illustrated by the broken lines contained in Figure 3.

When the angle of incident light is greater than a critical angle of incidence, "θ_{C}", losses of incident light due to reflection are minimized. This critical angle can be expressed as θ_{C} = sin⁻¹(n₂/n₁), where θ_{C} is the angle of incidence that will cause incident light to become trapped in the waveguide, and n₂ and n₁ are the indices of refraction for a first material and a second material that define a boundary across which the light is attempting to travel (e.g. - air and test strip material).

In order to further minimize such losses, the coupling edge of the waveguide may be treated with an anti reflective coating or an index-matching medium. For example, one or more thin layers of silicon oxide may be applied to the coupling edge. A thickness of approximately 200nm is an exemplary thickness.

Light trapped inside the waveguide propagates by total internal reflection (TIR) until it encounters the assay pad 20 which is affixed to or in communication with a surface of the waveguide, as illustrated in Figure 3. The assay pad 20 is provided with a surface in communication with the reflected light inside the waveguide, which is light scattering. Light impinging on this surface 34 of a test pad 20 is scattered at all angles. As illustrated by the dotted lines in Figure 3, some of the light is scattered at a large angle relative to the normal of surface 34. This light is also trapped inside the waveguide. However, some of the light is scattered at a relatively small angle compared to the normal of surface 34, as illustrated by the solid double arrow lines of Figure 3, and is consequently transmitted outside of the waveguide and received by the detector 24.

Another arrangement 400 of the type described above, is also illustrated in Figure 4. As illustrated in Figures 3 and 4, arrangements of this type (300, 400) are such that light provided by the light source 22 is incident upon the waveguide at a relatively small angle to the surface thereof, a larger amount of this incident light is transmitted through the waveguide, when compared with arrangements wherein the incident light is provided at a relatively steep angle to the incident surface. Thus, when the light source is an LED-type source, less power is required in order to provide the necessary amount of incident light to perform the assay, and power consumption can be minimized.

The light source 22 can be closely aligned with the edge of the waveguide 32, and thus may provide for a compact configuration. In addition, the detector element 24 can be positioned in close proximity to the waveguide 32, assay pad 20 and light source 22 (Figure 4), thereby enabling it to be more protected from stray light sources.

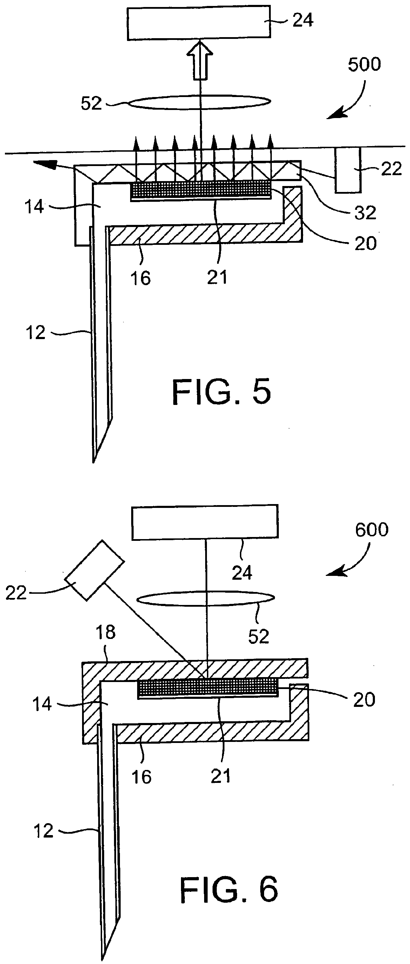

In certain instances, it may be desirable to provide an arrangement 500 as depicted in Figure 5. In this arrangement 500, an imaging lens 52 is provided and disposed such that it captures and focuses light reflected from the assay pad 20 toward the optical detector 24. The lens 52 may comprise a refractive or defractive element (Fresnel lens).

In Figure 5, the lens 52 is utilized in connection with an edge illumination-type arrangement.

Although the lens 52 is illustrated as being disposed at some distance from the waveguide 32 which forms the upper portion of the reaction chamber 14, the lens member may also be integrated into either the surface of the detector 24, or into the upper member (e.g. - 18, Figure 1) or waveguide 32.

Figure 6 depicts an alternative arrangement 600 which comprises a diffuse reflection type arrangement which incorporates an imaging lens member 52, as described above.

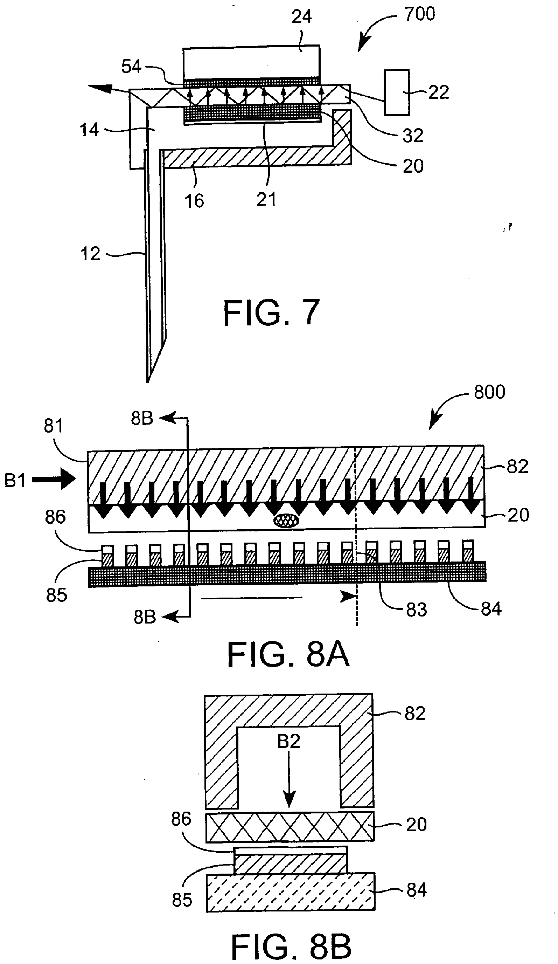

Another arrangement 700 is depicted in Figure 7. The arrangement 700 includes an edge-illumination type arrangement including waveguide 32. An imaging lens 54 is also included. In this arrangement, the imaging lens is integrated into either the waveguide 32 or the detector 24.

Figures 8a and 8b illustrate in further detail certain aspects of an embodiment of the present invention. Figures 8a and 8b illustrate an arrangement 80 which can be generally described as a vertical-flow assay.

The arrangement 800 includes a channel member 82 having an open "bottom", an assay pad 20 in communication with the open bottom of channel member 82, and an optical detector, preferably in the form of an array 84 of digital imaging devices 85. According to one preferred aspect of the invention, lens elements 86 are incorporated into the arrangement. Although lens elements 86 are illustrated as being in the form of discrete individual lenses in close proximity to the individual detector elements or pixels 85, it is within the scope of the present invention that alternative constructions are possible. For example, the lenses 86 could be spaced from the detectors 85, and/or a single integrated lens construction could be utilized instead of discrete lenses 86.

As previously noted, arrangements of the type depicted in Figures 8a and 8b permit the reliable and effective analysis of relatively small amounts of substances, which is not dependent upon a particular repeatable volume.

The channel member 82 is elongated. In other words, the substance being analyzed, such as a sample of body fluid is introduced at one end 81 of the channel member 82 and flows laterally or axially therethrough. The substance being analyzed then permeates the test pad 20 disposed thereunder. While the open channel member 82 defines a particular volume, it is not necessary to completely fill the channel member 82 in order to conduct an accurate analysis. For example, as illustrated in Figure 8A, a particular volume of substance to be tested may only partially fill channel member 82, for example, it may only fill channel member 82 up to the point defined along line 83. When conducting an analysis, the independent detector pixels 85 of the array 84 on the left-hand side of line 83 are activated and will take a reading from the analysis side of the test pad 20 once the analyte present in substance under analysis reacts with the chemicals contained in the test pad 20. Those individual detector elements of the array which are on the left-hand side of line 83 will generate a signal which is indicative of the presence of this reaction. Those individual detector elements 85 of array 84 which are on the right hand side of line 83 will not generate such a reading. Thus, the readings or data generated by each individual detector element 85 of the array 84 is taken and analyzed, such as by averaging, to generate an overall reading of the presence and/or concentration of a particular analyte contained in the substance under analysis.

Moreover, since the volume of the channel member 82 is known, and the length of the detector element contained in the array 84, as well as any correction due to the presence of lens elements 86 are known, the volume of the sample under analysis can be estimated based upon the number of pixels which detect the reaction between the analyte and the chemical reagents contained in test pad 20. An exemplary, non-limiting embodiment will now be described to further illustrate the principles of the present invention.

The time between the readings taken by the detectors 85 of the array 84 can also be monitored by the electronics of the arrangement. The specific means of implementing this monitoring being well within the skill of those in the art. Thus, the arrangement of the present invention can also analyze the kinetics of the reaction between the analyte and the chemical reagents, thereby offering the possibly to provide additional useful information that can be used to provide an insightful analysis of the sample.

An arrangement 80 of the type depicted in Figures 8a through 8b can advantageously be utilized to analyze very small volumes of a substance to be tested. For example, the arrangement can be utilized to assay 1-500 nanoliter (nL) volumes. The substance to be tested may include whole blood, interstitial fluid, or combinations thereof.

Assuming a nominal 250 nanoliter maximum volume, a sample of body fluid is introduced along direction B₁ from the left hand side 81 of channel number 82 through any suitable means, such as conduit 14 (see, e.g. - Figures 1 through 2). The sample is extended laterally into the elongated channel member 82. (In alternate embodiments, the volume of channel member 82 is 300 nanoliters.) The sample is extended over a relatively long length, which can be on the order of a few millimeters. According to one specific example, the channel is approximately 250 micrometers (width) x 140 micrometers (height) x 7,000 micrometers (length). The sample of body fluid then flows vertically along direction B₂ through the open bottom portion of channel 82 and into the assay pad 20. A color change in the assay pad 20 may be produced by a chemical reaction triggered by the presence of a specific analyte contained in the sample of body fluid, such as glucose. This color change can be detected on the opposite of the assay pad from which the sample of body fluid is introduced. This color change is detected by a suitable detection device, such as the optical detector array 84, which may be composed of a plurality of digital imaging detectors 85. According to one aspect, these digital detectors comprise either a CMOS, CCD, or photo diode array or infrared, ultraviolet or fluorescent sensors. It is to be understood, however, that other sensors may be used, all keeping within the scope of the present invention.

As a sample body fluid, such as whole blood, flows vertically through the test pad 20, red blood cells can be filtered or blocked from the opposite side of the test pad 20, which is generally viewed as being preferable so as to enable a more accurate reading of the levels of analyte present in the sample. The chemical reagents and dye products utilized in test pad 20 can be chosen from any number of well know substances. In any event, these chemistries should provide a discernable and readable reaction over an appropriate range of analyte concentration levels. In the case where the device is to be utilized to monitor the concentration of glucose contained in samples which comprise mainly whole blood, these chemistries should be able to produce reactions which can then be detected to indicate a glucose concentration ranging from, for example, 40-500 mg/dL.

According to a preferred aspect, the detector array 84 is formed from a plurality of individual CMOS-type detectors 85. CMOS detectors are less costly than other types of digital imaging devices. As described above, due to the time-dependent aspect of the arrangement 80, which may include CMOS detectors 85, various "clock" or time-driven electronic devices may be incorporated. Data can be taken from the detectors 85 and fed to such devices (directly or from storage) thereby enabling the monitoring and interpretation of the kinetics and other aspects of the reaction between the analyte and the reagent.

By the utilization of an array 84 of individual detectors 85, uniformity and accuracy of the assay is also promoted. For example, readings are taken and utilized from multiple individual detector elements, or pixels, are less susceptible to variations in uniformity of the materials of the assay pad, or other localized abnormalities, which can be accounted for and corrected by the interpretation and manipulation of the data generated by the individual pixels 85 of the detector array 84. For example, statistical analyses and associated algorithms may be used to correct for such localized defects, and therefore improve the accuracy of the overall reading generated by the device.

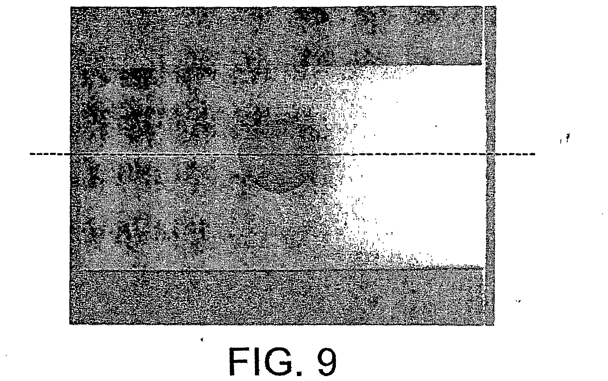

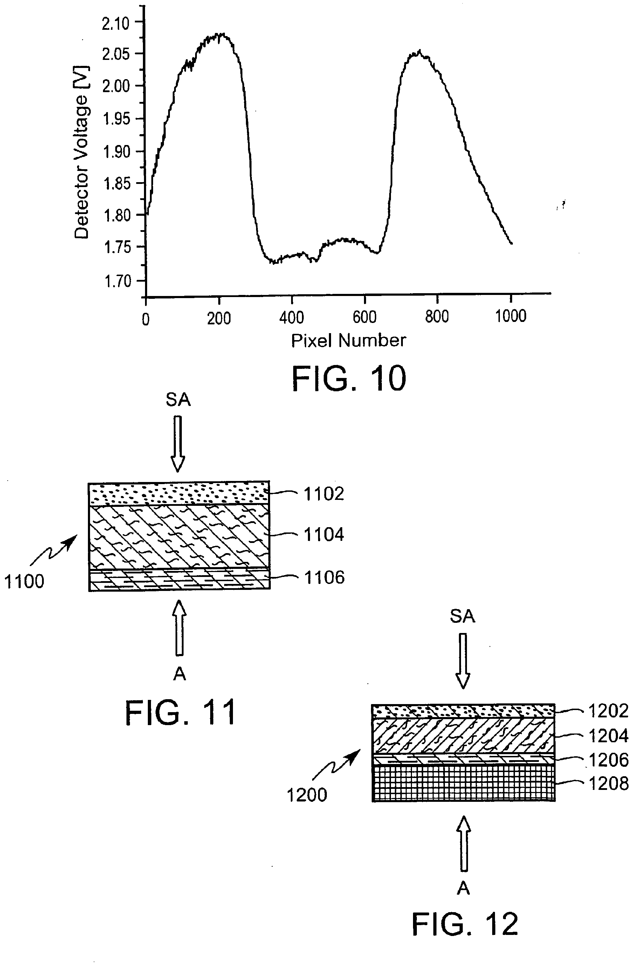

Figures 9 and 10 represent a scan performed by an arrangement 80 such as the one depicted in Figures 8a and 8b. Figure 9 is a picture of a test spot formed on a commercially-available assay pad. Figure 10 is a plot of the signal generated by an array of detector elements 84 as they are scanned along the dashed line contained in Figure 9. As illustrated in Figure 10, the lower voltage readings correspond to the area occupied by the test spot of Figure 9 along the dashed line.

Figure 11 is a schematic illustration of one possible construction for the assay pad 20. The assay pad construction 1100 has a sample application side SA as well as an analysis side A. According to one construction, the assay pad 1100 is composed of at least 3 constituent components: a blocking component 1102; a membrane component 1104; and a chemical reagent component 1106. When the sample to be analyzed is composed at least in part of whole blood, red blood cells are separated from the plasma, which is then transported to the reagent component 1106 which includes enzymatic chemistries selected to produce a chemical reaction with one or more analytes present in the sample.

The blocking component 1102 may include an agent to filter or pre-filter red blood cells. By way of example, the blocking component 1102 may include zirconium oxide (ZrO₂).

The membrane component 1104 can be formed of any suitable material. For example, the membrane may be a high light transmitting material, or optionally can be an opaque or substantially opaque material. Suitable materials may include styrene and/or nylon. The membrane component 1104 can be in woven or non-woven form. According to one aspect, the membrane is in the form of a woven mesh.

As indicated above, the reagent component 1106 is chosen according to its ability to react with the analyte under investigation. Any suitable reagent chemistry can be utilized. Specific examples of suitable material include a glucose oxidase (e.g. - from Biocatalysts product G575P), a soybean peroxidase (e.g. - from Organic Technologies, product 73930.1 medical diagnostic grade), an amino antipyrine hydrochloride (e.g. - from TCI, product A0257) and an Aniline derivative dye for a "Trinder" reaction (see, e.g. - P. Trinder, "Determination of Glucose in Blood Using Glucose Oxidase with an Alternative Oxygen Acceptor").

While the various constituent components of the assay pad construction 1100 are illustrated in Figure 11 as distinct layers, it should be understood that the present invention is not limited to such a construction. For example, the blocking component 1102 and/or the reagent component 1106 may be partially or entirely subsumed and contained within the membrane component 1104. Thus, it is entirely possible that a surface "layer" of the blocking component 1102 and/or reagent component 1106 may be present. Alternatively, the blocking component 1102 and/or the reagent component 1106 may be entirely subsumed, contained or impregnated within the membrane component 1104, such that no distinct blocking component "layer" or reagent component "layer" is present at all. As such, the present invention encompasses embodiments where more than one of the claimed (first, second, third, fourth) components are performed by the same physical element. Conversely, the present invention also encompasses embodiments where a plurality of different physical elements work together to perform the features of one of the claimed (first, second, third, fourth) components. The present invention also encompasses embodiments with no sample pad at all.

It should be noted, that not only with respect to the embodiment depicted in Figure 11, but as well as with all of the following alternative test strip constructions according to the present invention (e.g. - Figures 12-16), similar alternative constructions to that explicitly described above are comprehended within the scope of the present invention. For instance, the constituent components described herein are not limited to distinct "layers". Rather, one or more of the constituent components of the test strip constructions described herein may be partially, or entirely, subsumed, contained or impregnated within other constituent components of the test strip constructions and therefore not be present as distinct "layers".

With respect to the embodiment depicted in Figure 11, the blocking component 1102 and/or reagent component 1106 may be applied to the membrane component 1104 in any suitable manner. For example, the blocking component 1102 and/or reagent component 1106 may be coated upon the membrane 1104. An example of suitable coating techniques includes spin coating.

Figure 12 illustrates one possible alternative construction 1200 for the assay pad 20. According to one construction, the assay pad construction 1200 is composed of at least 4 components: a prefilter and reflective component 1202, a membrane component 1204, a reagent component 1206, and a mesh component 1208. With the above-described structure, in the case where the substance to be analyzed is a sample which is composed at least in part of whole blood, the red blood cells are separated from the plasma, so that components 1202 and/or 1204 retain the red blood cells without lysis. The plasma is then vertically transported to the reagent component 1206 which includes enzymatic chemistries. These substances may include any suitable material, which are generally known in the art. Specific examples of suitable materials include a glucose oxidase (e.g. - from Biocatalysts product G575P), a soybean peroxidase (e.g. - from Organic Technologies, product 73930.1 medical diagnostic grade), an amino antipyrine hydrochloride (e.g. - from TCI, product A0257) and an Aniline derivative dye for a "Trinder" reaction (see, e.g. - P. Trinder, "Determination of Glucose in Blood Using Glucose Oxidase with an Alternative Oxygen Acceptor").

The component 1204 may comprise any suitable blood-filtering component or material. Some materials which have been used to separate red blood cells from plasma and samples of whole blood include glass fibers, mesh cloth, unwoven mats, asymmetric membranes, and porous polymers.

Regardless of what type of material is chosen for component 1204, the avoidance of spreading or lateral diffusion, which may occur with such materials as poly (ethersulfone) membranes, is desirable. An exemplary suitable material is, a hydrophilic poly(vinylidene fluoride) unwoven membrane. Such membranes are commercially available from the Millipore Corporation (e.g. - with 0.1 micron openings). Other suitable materials include unwoven membranes comprising a polyamide. Other materials may be incorporated to achieve various performance objectives, such as poly(acrylic acid) (PAA), hydroxyethyl cellulose, sodium alginate, and gelatin.

The pad 1200 may further include a component 1202, such as a prefilter and/or diffuse reflection component. Thus, the component 1202, when included, serves to improve separation of red blood cells from a sample containing whole blood, and/or increase the reflection of incident light upon the opposite side of the pad when undergoing optical analysis. Suitable materials for use as component 1202 include a zirconium oxide (ZrO₂), optionally combined with a polymer binder material. The component 1202 can be applied to the membrane, or component 1204, by using any suitable technique. One such suitable technique is spin coating. Spin coating promotes uniform distribution of the component 1202 onto and/or within the membrane component 1204. As previously explained, while components 1202 and 1204 have been illustrated as distinct layers, the component 1202 may be partially, or entirely contained within the membrane component 1204, thus effectively combining these two components into a single unitary member, rather than the illustrated distinct layers. When, according to the above-described alternative embodiment, the component 1202 comprises a prefilter and diffuse reflection material and a polymer binder, the presence of the polymer binder tends to form at least a thin layer or film over the membrane component 1204. This thin film may have any suitable thickness, for example, the component 1202 may partially permeate the membrane component 1204 and leave a thin film having a thickness on the order of less than 5 micrometers on top of the membrane component 1204.

The component 1206 comprises a reagent which may be formed from any suitable composition, such as the chemicals described above, and may optionally be provided in a binder of poly(acrylic acid), hydroxypropylmethylcellulose (HPMC) or similar polymers that reduce lateral spreading of the substrate or sample undergoing analysis.

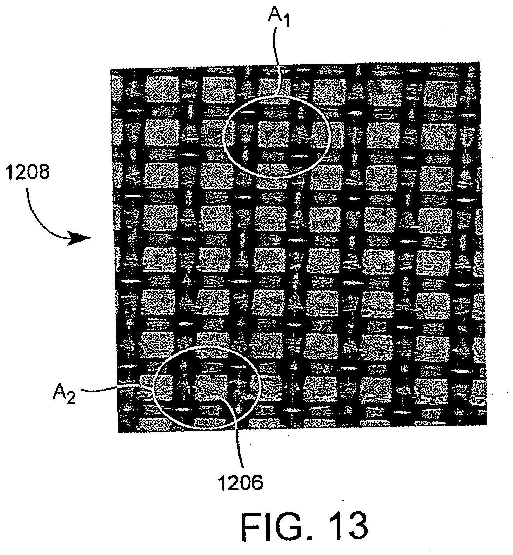

The mesh component 1208 and the reagent 1206 component may also be integrated into a single layer or constituent element of the test pad 20. For example, reagent material 1206 can be coated onto the mesh component 1208. Any suitable coating technique may be utilized. A spin coating technique is one such technique for providing the reagent on the mesh component 1208 which allows for precise and relatively thin coating of the reagent component 1206 to be applied to the mesh component 1208. According to one construction, this "coating" is entirely absorbed and contained within mesh component 1208. The mesh component 1208 should be permeable to oxygen in order to promote the reaction of the analyte with the chemical reagent component 1206. According to one aspect, the mesh component 1208 can be formed from a high reflectance woven mesh, such as nylon. Thus, according to one aspect, mesh component 1208 is formed from an open polyamide or nylon mesh material. This nylon mesh material having a reagent incorporated therein is believed to increase the sensitivity of the assay. The open structure of the nylon mesh, in conjunction with the controlled amount of the reagent component material 1206 incorporated therein, increases the diffuse reflection of light incident thereon. This increased diffuse reflection is believed to be caused by the multiple reflections and increased path lengths provided by the open nylon mesh structure. According to one exemplary embodiment shown in Figure 13, the mesh component 1208 has openings have a dimension which is on the order of 68 microns, and a filament diameter which is on the order of approximately 50 microns. The region indicated by circle A₁ illustrates the absence of reagent material within the mesh, wherein the reagent material has been consumed by chemical reaction with the analyte. Circle A₂ indicates an area of the mesh in which the reagent material is still disposed thereon.

The cylindrical fibers of the nylon mesh may scatter the light onto the external exposed layer or surface thereby introducing multiple reflection and increased path links. The scattering of incident light is largely dependent upon a refractive index of the cylinders which form the filaments of the mesh. The refractive index of nylon is relative large, on the order of 1.53, and this is beneficial. Moreover, the above-described application of the reagent component 1206 to the mesh component 1208 can simplify manufacture.

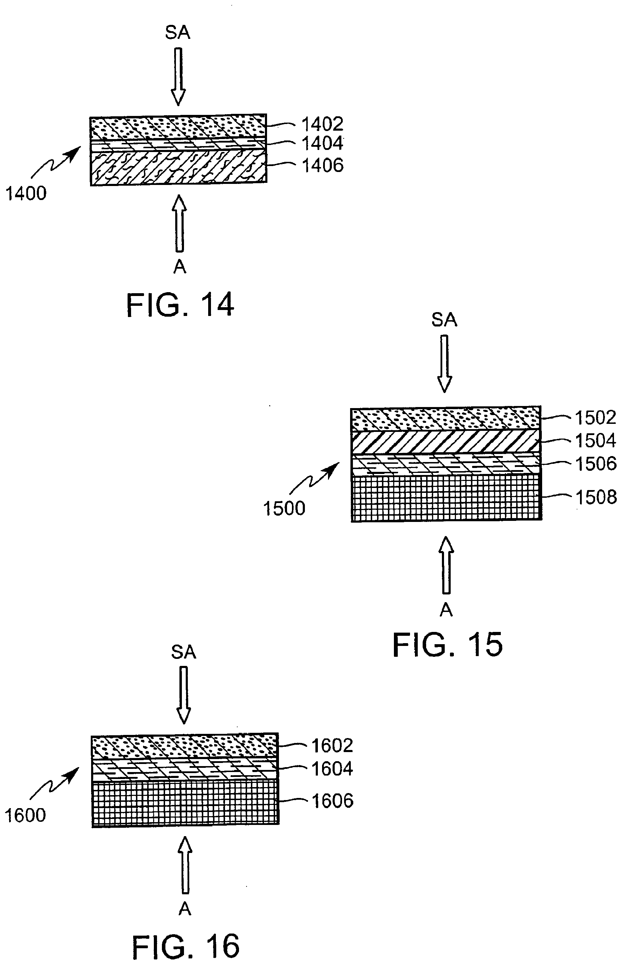

Another alternative test pad or test strip construction 1400 is illustrated in Figure 14. According to one aspect, the test pad construction 1400 includes three basic components: a filter and/or diffuse reflective component 1402, a chemical reagent component 1404, and a membrane component 1406. As previously discussed, a sample to be analyzed is applied to the sample application side SA of test pad 1400. When the sample contains, at least in part, whole blood, red blood cells are separated from the plasma by component 1402 and are transported to the reagent component 1404, which may be partially or entirely impregnated it the membrane component 1406.

The component 1402 may be formed from any suitable construction that provides filtration and/or diffuse reflection functions. For example, suitable constructions have been described above. According to one specific example, the component 1402 may comprise a zirconium oxide material combined with a permeable polymer binder material, such as HPMC, that limits lateral diffusion. Another suitable example includes a zirconium oxide material combined with a permeable polymer binder such as vinyl butyral. The zirconium oxide and polymer binder are combined in any suitable proportions, such as 20:80.

The reagent component 1404 includes at least one chemical reagent chosen to provide a suitable reaction with the analyte(s) under investigation. Any suitable reagent can be chosen. For example, the reagent may comprise a glucose oxidase, soy bean peroxidase, amino antipyrine hydrochloride, and/or aniline derivative dyes. The chemical reagent may optionally be combined with a binder component. When present, the binder component is preferably formed from a material which reduces the tendency for lateral spreading of the sample within the test pad 1400. Suitable examples binder material examples include HPMC and PAA.

A suitable membrane component 1406 can also be provided. According to one embodiment, the membrane component 1406 can comprise a fibrous unwoven material. One specific example includes a cellulose acetate unwoven fibrous membrane material.

Both the filter/diffuse reflective component 1402, as well as the reagent component 1404 can be applied to the membrane component 1406 by any suitable technique. According to one example, the reagent component 1404 is applied to the membrane component 1406 by a coating technique, such as spin coating. Reagent component 1404 may be partially, or entirely impregnated within the membrane component 1406. Thus, a thin film or layer of reagent component 1404 may be present on the top surface of the membrane component 1406 (i.e. - the side closest to the sample application side SA of the test pad 1400). When present, this thin top layer can be on the order of a few microns in thickness. Alternatively, the reagent component may be entirely subsumed, contained or impregnated within the membrane component 1406, such that no distinct layer is present on the top surface of the membrane component 1406 (i.e. - the surface of membrane 1406 which is closest to the surface application side SA of the pad 1400).

The filter/diffuse reflective component 1402 can then be applied subsequent to application of the reagent component 1404. The component 1402 can also be applied by any suitable technique, such as spin coating. According to one specific example, a filter/diffuse reflective component comprising a zirconium oxide and hydrophobic permeable polymer binder material is spin coated at approximately 1000 rpm onto the membrane/reagent components 1406/1404. The filter/diffuse reflective component 1402 may be partially impregnated within the membrane 1406, leaving a thin film layer at the surface application side SA of the combined membrane and reagent components 1406 and 1404. When present, this thin surface layer can be a few microns in thickness, for example, on the order of less than 5 microns in thickness. Alternatively, the filter/diffuse reflective component 1402 may be entirely subsumed, contained, or impregnated within the combined reagent and membrane components 1404 and 1406.

An additional test pad construction 1500 consistent with the principles of the present invention is illustrated in Figure 15. According to one aspect, the test pad construction 1500 is comprised of at least four basic components: a filter/diffuse reflective component 1502, a permeable material component 1504, a reagent component 1506, and a membrane component 1508. The general functionality of the test pad 1500 and the functionality of the individual components thereof are similar to that previously described.

The component 1502 may comprise any suitable filter and/or diffuse reflective material. For example, the component 1502 may comprise a zirconium oxide material with or without a polymer binder. When included, the polymer binder is preferably a permeable material. Examples of suitable permeable polymer binders include HPMC and an acrylic resin, such as Elvacite®.

The component 1504 is preferably comprised of a suitable permeable material which can be hydrophobic. The hydrophobic material can comprise any suitable form. According to one example, the component 1504 may comprise a film of a hydrophobic polymeric material, such as PAA. The film can be formed by any suitable technique. For example, a film of PAA is formed by a fast evaporating solvent/PAA mixture. According to one specific example, the mixture may contain 10% by weight (relative to the weight of the solvent) of PAA.

The component 1506 may comprise a suitable reagent material. The reagent material may be combined with a binder. The reagent component may be chosen from any suitable substance or combination of substances, as described above. When present, the binder material comprises a permeable hydrophobic binder material. One suitable example is HPMC.

The membrane component 1508 can be formed from any suitable material. According to one possible construction, the membrane component 1508 is formed from an unwoven fibrous mat. For example, the reagent component 1506 can be spin coated upon the membrane component 1508. As discussed above, the reagent component 1506 may be partially, or entirely, impregnated within the membrane component 1508. Thus, the reagent component 1506 may or may not comprise a thin surface layer formed on top of the membrane component 1508 (the top side being closest to the surface application side SA of the test pad 1500).

The hydrophobic material component 1504 can then be applied. According to one possible technique, the hydrophobic material can be formed as a slurry or mixture with a fast evaporating solvent, such as methanol. The mixture is then coated onto the combined membrane and reagent components 1508 and 1506. Subsequent to evaporation, a thin film of the hydrophobic material is left on top of the combined reagent/membrane component.

The component 1502 may then be applied to the previously described combined components. According to one example, a combination of filter/diffuse reflective material, e.g. - ZrO₂, and a polymer binder material, such as the ones described above, may be combined in the form of a slurry or mixture and then coated upon the previously combined components. According to one specific example, a slurry containing up to 10% by weight of ZrO₂ may be formed and then spin coated onto the previous combined constituent components 1504, 1506, and 1508. As previously discussed, the component 1502 may be partially or entirely impregnated or subsumed within the previously combined components. Thus, the component 1502 may be indistinguishable from the previously combined components or, may form a thin film or layer on top of the previously combined components.

Yet another alternative test pad construction 1600 formed according to the principles of the present invention is illustrated in Figure 16. The test pad construction 1600 illustrated therein is formed from at least three basic constituent components: a filter/diffuse reflective component 1602, a permeable hydrophobic material component 1604 and a combined reagent/mesh component 1606. The basic functionality of the individual as well as combined constituent components of the test pad construction 1600 is similar to that previously discussed.

The filter/diffuse reflective component 1602 can be formed from any suitable material, and comprise any suitable construction. According to one possible embodiment, the component 1602 comprises a zirconium oxide material, which may optionally be combined with a binder material. When present, the binder material is preferably a permeable hydrophobic binder material such as an acrylic resin. Such material is being commercially available, e.g. - sold as Elvacite®.

Similarly, the permeable hydrophobic material component 1604 can be comprised of any suitable material, and take any suitable form. By way of example, the permeable hydrophobic material can comprise a film or layer. According to one specific example, the permeable hydrophobic material may comprise PAA.

The combined reagent/mesh component 1606, as implied is preferably the combination of two distinct parts or constituents. Namely, a reagent material such as those previously described, as well as a mesh material constituent. Preferably, these constituents are combined into a single, integral component 1606. The mesh material can be formed of any suitable material and take any suitable form. For example, the mesh material can be formed from a woven polyamide material, such as a woven nylon mesh. The mesh may have openings of any suitable dimensions, such as either 30 microns openings or 41 micron openings.

The above-described constituents can be applied to one another or combined in any suitable manner.

According to one specific example, the reagent and the mesh component are combined or integrated by soaking the mesh material in a slurry formed from the reagent material. For example, a slurry or mixture can be formed which comprises 20% by weight HPMC binder material plus a suitable reagent chemistry. The mesh material is soaked in this liquid slurry. The mesh material is then removed and dried, thereby leaving an integrated mesh/reagent component 1606.

The hydrophobic component 1604 can be similarly be applied by any suitable technique. For example, as described above, the hydrophobic material can be applied as a film formed from a fast evaporating solvent, like methanol. According to one specific example, a hydrophobic material, such as PAA is combined with a solvent to form a slurry. According to one embodiment, the slurry may comprise 10% by weight of the PAA material. The solvent is then applied to the combined reagent/mesh component 1606. Upon evaporation of the solvent, a thin film or layer of the hydrophobic material is left behind, thereby forming the hydrophobic material component 1604.

The filter/diffuse reflective component 1602 may similarly be applied by any suitable technique, such as those previously described. According to one aspect example, a zirconium oxide material is added to a hydrophobic binder to form a slurry in an evaporative solvent such as toluene. According to this specific example, the slurry contains approximately 10% by weight of acrylic resin such as Elvacite®. This slurry is then coated onto the combined hydrophobic and reagent/mesh components 1604 and 1606. Any suitable coating technique can be utilized, such as the aforementioned spin coating technique.

According to one possible construction, the filter/diffuse reflective component 1602 is provided in the form of a thin layer or film on top of the combined hydrophobic material and reagent/mesh component 1604 and 1606.

A further aspect of the present invention provides effective and efficient constructions and techniques that allow for the reliable collection and transport of relatively small quantities of body fluids, such as blood.

As used herein, the term "body fluid" includes at least the following components: blood, interstitial fluid, and combinations thereof. The term "skin penetration member" includes hollow needles and solid lancets, as well as multiple component constructions, such as concentric needles or a concentric hollow tubular collection member and needle arrangement. The term needle includes, but is not necessarily limited to, so-called "microneedles" or small gage needles. For example, such microneedles can have an outer diameter on the order of 40 - 200 µm and an inside diameter on the order of 25 - 160 µm. Similarly, these "microneedles" can be of a small gage, such as on the order of 34 or 36 gage needle.

Further, it should be understood throughout the following description that the sample of body fluid can be obtained from either within the wound beneath the surface of the skin and/or off the surface of the skin itself.

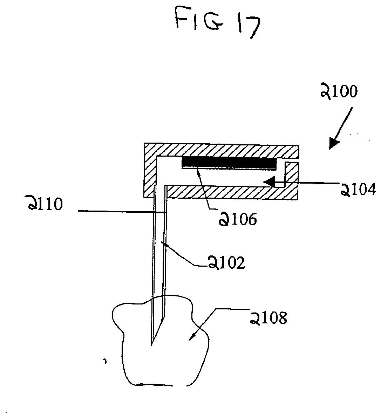

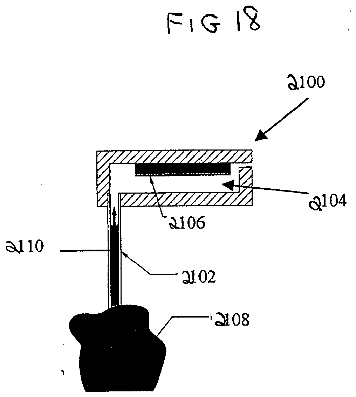

Figures 17 and 18 are illustrative of an arrangement 2100 for the collection an analysis of a sample of body fluid 2108, such as blood. The illustrated arrangement 2100 generally comprises a skin-penetration member 2102, an analysis chamber 2104, an analysis component 2106, and an inner bore 2110 of the skin-penetration member 2102. As illustrated in Figure 2, body fluid 2108 can be drawn up through the inner bore 2110 in the direction of the heavy arrow via capillary forces. As can be appreciated, the embodiments of the present invention illustrated in Figures 17 to 23 operate in the same manner as described above with reference to Figures 1 to 16.

As illustrated in Figures 17 and 18, a continuous and uninterrupted path is defined by the inner bore 2110 between the point of body fluid acquisition to the analysis chamber. It should be understood that this principle of the present invention is not necessarily reliant upon all of the details of the arrangement 2100, and thus may clearly have utility in other contexts.

According to further examples, the analysis chamber 2104 is vented in order to facilitate capillary flow and preventing the build-up of a back pressure within the analysis chamber 2104 that would impede flow of the body fluid 2108.

According to another example, the inner bore 2110 can be modified in a manner that promotes capillary action. For example, the inner bore 2110 can be coated with a substance that promotes capillary action.

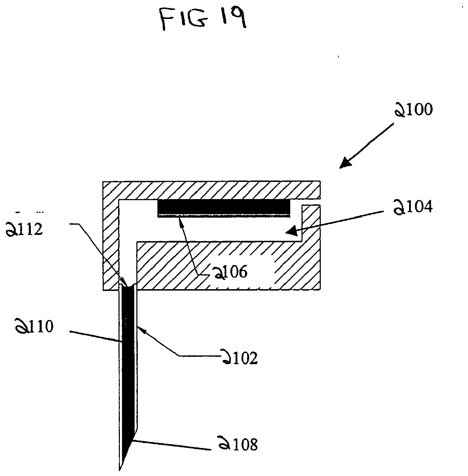

Figure 19 illustrates an arrangement similar to that previously illustrated in Figures 17 and 18, however, the arrangement 2100 of Figure 19 does not provide a continuous uninterrupted path from the point of body fluid acquisition to the analysis chamber 2104. Instead, the arrangement 2100 of Figure 19 includes a capillary break at 2112.

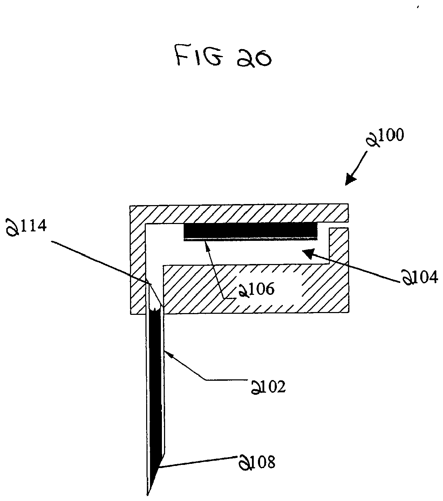

The arrangement depicted in Figure 20 also includes a capillary break 2114, but the capillary break 2114 has a construction designed to minimize the adverse effects on the capillary flow of body fluid 2108. Thus, the arrangement of Figure 20 is considered an improvement, at least in this respect, over the arrangement of Figure 19.

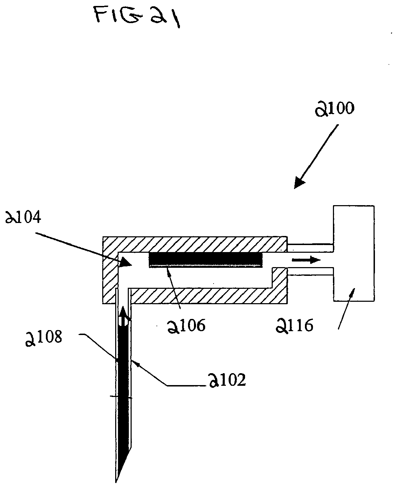

Figure 21 illustrated another arrangement 2100. The primary distinction between the arrangement of Figure 21 and the previously described arrangements is the use of an applied vacuum via a vacuum generation device 2116 in order to assist body fluid collection and transportation.

It should be appreciated that this example can find application in arrangements other than that illustrated in Figure 21.

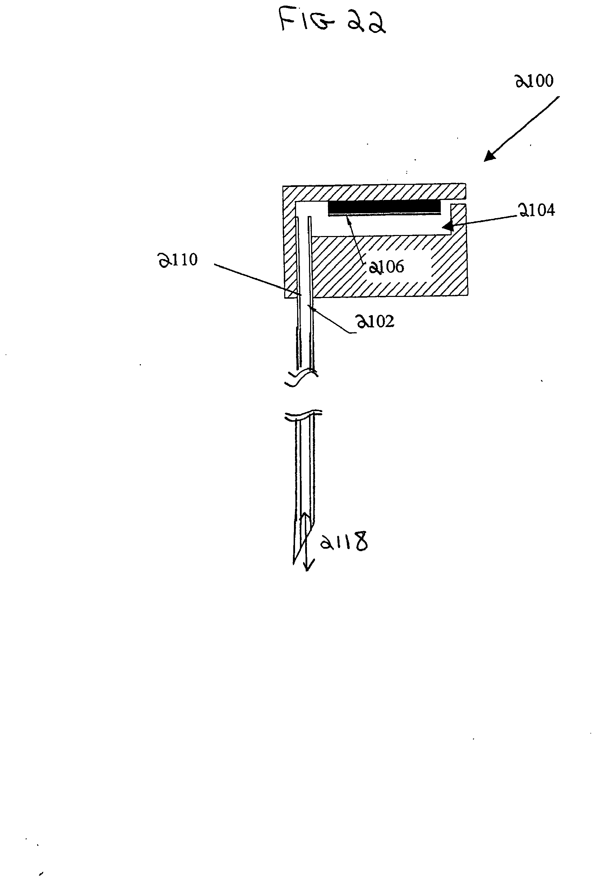

As illustrated in Figure 21, the inner bore 2110 of the illustrated example provides a continuous and uninterrupted path from the point of body fluid acquisition to the analysis chamber 2104. The vacuum generating device facilitates the flow of body fluid along this uninterrupted path. However, it should be noted that the application of a vacuum can also be beneficial in arrangements such as those illustrated in Figures 19 and 20 that have a capillary break (e.g. - 2112, 2114). The arrangement depicted in Figure 22 is similar to the previous arrangements except
that the analysis chamber 2104 is static, while the skin penetration member 2102 is moveable along the direction indicated at double-headed arrow 2118 relative thereto. This construction necessitates a moveable seal between the skin penetration member 2102 and the analysis chamber 2104 designed to maintain the capillary flow.

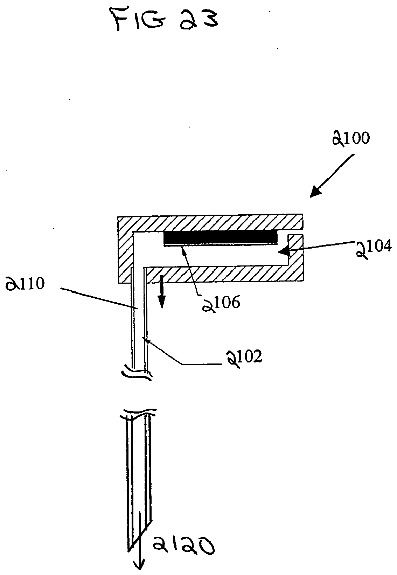

Unlike the previously described arrangement, the skin-penetration member 2102 and the analysis chamber 2104 are relatively fixed, as illustrated in Figure 23. Although these two members are fixed relative to one another, and thus move together upon insertion of the skin-penetration member, the members can be moveable via connection to a hinge or translation stage (not shown).

Thus, in various embodiments, the present invention provides arrangements for the detection of the presence and/or concentration of an analyte in a sample of bodily fluid include diffuse transmission, diffuse reflection and edge or waveguide illumination arrangements. A vertical flow assay arrangement and/or technique is also disclosed, and includes a detection component that can be in the form of an array of optical detection elements. A number of assay pad constructions are described which may include at least one or more of the following components: a prefilter component, a reflective component, a membrane component, a reagent component, a mesh component, and a component to prevent lateral spreading. It is to be understood that in alternate embodiments, the present invention can be operated without an assay pad.

The present disclosure also describes: a system for effective and efficient techniques and constructions that allow for quantitative measurement of the concentration of an analyte using algorithms. As will be shown such algorithms may be based on peak average, peak area, or peak value with respect to the measurement of the concentration of glucose levels present in a nano liter-sized sample of body fluid using a CMOS-based detector device. In preferred examples, the algorithms are constructed to select data from the measurement that results in improved assay statistics.

According to an exemplary aspect of the present invention, three algorithms can be utilized for quantitative glucose measurements using CMOS data.

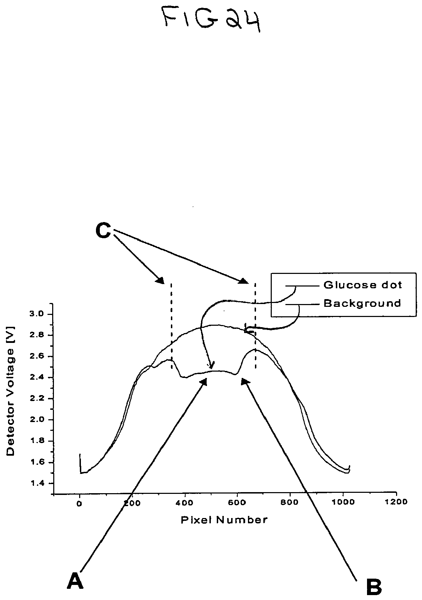

According to an illustrative embodiment, glucose measurements were made in a diffuse reflection mode where a narrow wavelength LED emits light onto an assay device and signal is recorded with a CMOS array. In the presence of glucose, a color develops on the assay device and the diffuse reflectance signal detected by CMOS is reduced by the absorption of light. The level of reduction depends upon the glucose concentration. Figure 24 shows typical curves for CMOS detection. In this figure, the background line shows the CMOS signal before glucose was delivered to the assay device. The glucose dot line shows the signal after glucose was delivered to the assay device.

The width of color is indicated by the length of the absorption shown by the glucose dot line. Note the variation in diffuse reflectance from A to B is due in part to the baseline variation shown in the red trace. C indicates the approximated width of the assay. The width can be converted into number of pixels. This assay may be corrected for background, assay width and edge effects to improve the assay statistics. According to the present disclosure, three methods are available for evaluating the CMOS data. The data obtained from these algorithms correlate with glucose concentration.

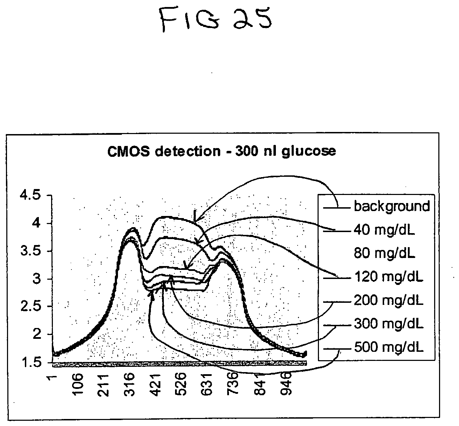

Figure 25 shows an overlay of CMOS glucose assays. In this case, 300 nanoliters of each glucose concentration was delivered to an assay device. The actual glucose concentration is determined by measurement using a Yellow Spring Instrument that is well established as reliable and accurate. Each curve in this graph represents the average of two measurements.

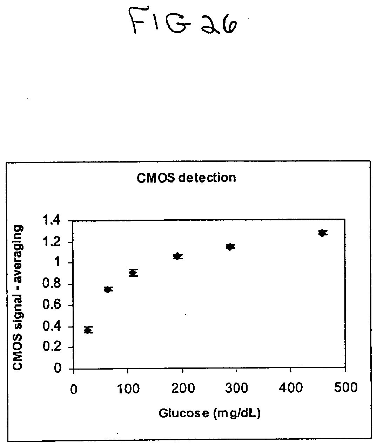

Three algorithms were utilized to evaluate the accuracy of above measurements. The first is based on "peak averaging" where the average value was taken within the flat part of the absorption region, C in Fig. 24. In the above case, it is from pixel 450 to pixel 550. This average value then is subtracted from the background. We plot this net value against the glucose concentration as shown in Figure 26. The error bars in this curve are the standard deviation.

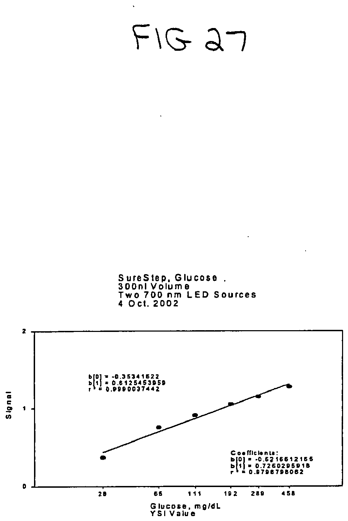

The data in Fig. 26, may be plotted with the logarithm of the glucose concentration that is linear with signal, as illustrated in Fig. 27. The least squares coefficients are determined to evaluate the accuracy of the predicted, least squares value in relation to measured values. These least square coefficients are shown on Fig. 27 for all points and for the set without the "low" point.

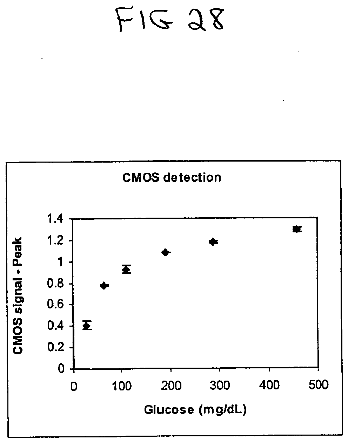

The second exemplary algorithm is based on "peak value" where the minimum value between pixel 450 and pixel 550 was used as our signal; this value is then subtracted from the background. We plot this net value against the glucose concentration as shown in Figure 28.

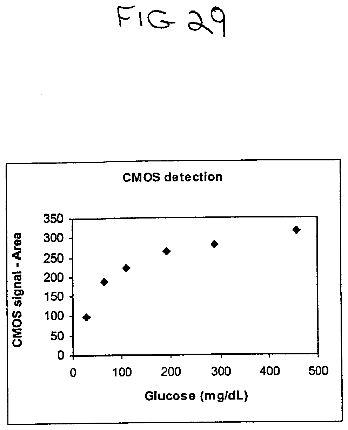

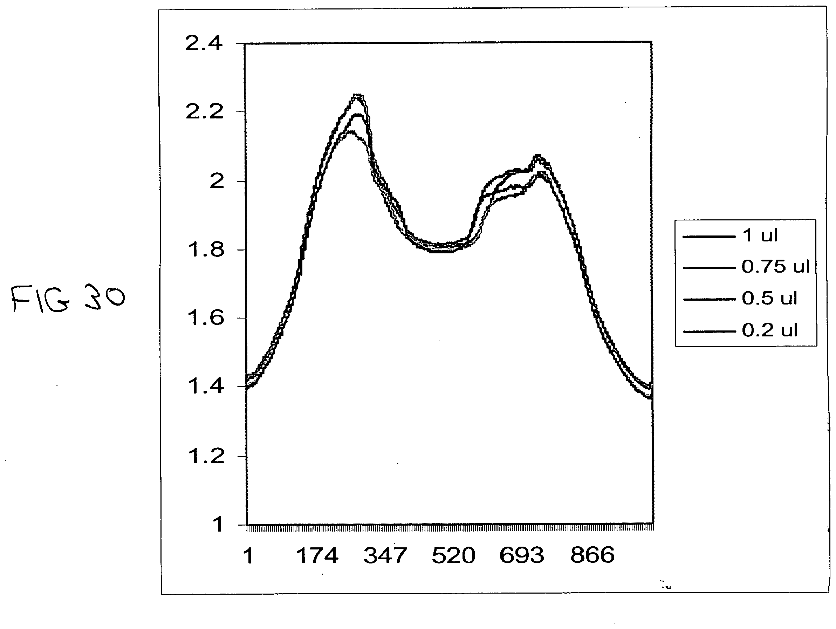

The third exemplary algorithm is based on "peak area". At each pixel we take the difference between the background and the signal and then we sum up this net value from pixel 450 to pixel 550. We plotted this against glucose concentration. The result is shown in Figure 29. Lastly, Figure 30 is a plot of glucose concentration versus detector signal for four different test volumes illustrating the volume independence advantage of the present invention.

The following table summarizes the analyzed results for all three methods. The "predicted glucose" is the calculated glucose value using logarithmic fit function. The "r-square" is the best-fit r square value using logarithmic fit. The "error" is the percentage error in compares our calculated glucose with YSI reading.

| Peak Average | | | | | | |
|---|---|---|---|---|---|---|
| Glucose (mg/dL) | log(glucose) | CMOS | predicted Log(glucose) | predicted glucose | error | r-square |
| **28** | 1.447158031 | 0.366183 | 1.360609298 | **22.9** | **-22.05%** | |
| **65** | 1.812913357 | 0.751 | 1.890638128 | **77.7** | **16.39%** | |
| **111** | 2.045322979 | 0.904599 | 2.10219827 | **126.5** | **12.27%** | |
| **192** | 2.283301229 | 1.053475 | 2.307253485 | **202.9** | **5.37%** | |
| **289** | 2.460897843 | 1.148262 | 2.437808873 | **274.0** | **-5.46%** | |
| **458** | 2.660865478 | 1.274866 | 2.612187288 | **409.4** | **-11.86%** | 0.9799 |

| Peak Area | | | | | | r-square |
|---|---|---|---|---|---|---|
| Glucose (mg/dL) | log(glucose) | CMOS | predicted Log(glucose) | predicted glucose (mg/dL) | error | |
| **28** | 1.447158031 | 97.7975 | 1.368763277 | **23.4** | **-19.78%** | |
| **65** | 1.812913357 | 188.6775 | 1.890552334 | **77.7** | **16.37%** | |
| **111** | 2.045322979 | 221.7315 | 2.080332434 | **120.3** | **7.74%** | |
| **192** | 2.283301229 | 264.0705 | 2.323422518 | **210.6** | **8.82%** | |
| **289** | 2.460897843 | 281.6805 | 2.424530631 | **265.8** | **-8.73%** | 0.9823 |
| **458** | 2.660865478 | 316.2675 | 2.623112476 | **419.9** | **-9.08%** | |

| Peak Value | | | | | | r-square |
|---|---|---|---|---|---|---|
| Glucose (mg/dL) | log(glucose) | CMOS | predicted Log(glucose) | predicted glucose (mg/dL) | error | |
| **28** | 1.447158031 | 0.406545 | 1.271765735 | **18.7** | **-49.76%** | |
| **65** | 1.812913357 | 0.779545 | 1.842713232 | **69.6** | **6.63%** | |
| **111** | 2.045322979 | 0.927545 | 2.069255403 | **117.3** | **5.36%** | |
| **192** | 2.283301229 | 1.080545 | 2.303451025 | **201.1** | **4.53%** | 0.9959 |
| **289** | 2.460897843 | 1.176545 | 2.450397297 | **282.1** | **-2.45%** | |
| **458** | 2.660865478 | 1.297045 | 2.634845484 | **431.4** | **-6.17%** | |

While the present invention has been described by reference to the above-mentioned embodiments, certain modifications and variations will be evident to those of ordinary skill in the art. Therefore, the present invention is limited only by the scope of the appended claims.

## Claims

1. A device for conducting an assay to determine the concentration of an analyte in a sample of bodily fluid, the device comprising:
a channel member (82) having an open bottom, a first opening and a second opening, wherein the first opening is at one end of the channel member (82) for introduction of the sample to the channel member (82) and allowing flow of the sample therethrough;
an assay pad (20) in communication with the open bottom of the channel member (82) and covering the channel member (82), the assay pad (20) comprising a reagent adapted to produce a chemical reaction when exposed to the analyte, the chemical reaction producing a color change in the assay pad (20); and
a linear array (84) of CMOS optical detectors (85) disposed relative to the assay pad (20) so as to detect the color change, wherein the channel member (82) defines a length, and the linear array (84) of CMOS optical detectors has a plurality of CMOS sensors that define a length of the array (84) that is commensurate to the length of the channel member (82).

2. The device of claim 1, wherein the analyte comprises glucose.

3. The devices of claim 1, wherein the bodily fluid comprises whole blood.

4. The device of claim 1, wherein the channel member (82) is a microchannel.

5. The device of claim 1, further comprising a light source (22) adapted to provide light incident upon the assay pad (20).

6. The device of claim 5, wherein the light source (22) is arranged relative to the channel member (82) and the assay pad (20) such that light transmitted from the light source is at least partially transmitted through the channel member (82) and the assay pad (20), the optical detectors (85) are arranged in a manner such that the transmitted light is received by the optical detectors (85).

7. The device of claim 5, wherein the light source (22) is arranged relative to the channel member (82) and assay pad (20) such that light is transmitted from the light source and at least partially reflected off the assay pad (20), and the optical detectors (85) are arranged relative to the channel member (82) and assay pad (20) to receive light reflected off the assay pad (20).

8. The device of claim 5, further comprising a waveguide (32) associated with the assay pad (20), the light source (22) is arranged such that light emitted therefrom is incident upon that waveguide (32), the waveguide (32) is arranged relative to the assay pad (20) such that light transmitted therein from the light source illuminates the assay pad (20), the assay pad (20) is constructed to at least partially reflect the light incident thereon, and the optical detectors (85) arranged to receive light reflected by the assay pad (20).

9. The device of claim 8, wherein the waveguide (32) is arranged to illuminate a lateral edge of the assay pad (20), and the optical detectors (85) are arranged to receive reflected light via the waveguide (32).

10. The device of claim 8, wherein, at least one side of the waveguide (32) is provided with an anti-reflective coating.

11. The device of claim 1, further comprising a lens (86) disposed between the optical detectors (85) and the assay pad (20).

12. The device of claim 11, wherein the lens (86) is a refractive lens.

13. The device of claim 11, wherein the lens (86) is a diffractive lens.

14. The device of claim 11, wherein the lens (86) is integral with the optical detectors (85).

15. The device of claim 1, wherein the device further comprises a skin penetration member (12) for extracting the sample of bodily fluid from a wearer.

16. The device of claim 1, wherein the channel member (82) has a general shape of an elongated upside-down U when disposed in its operable position within the device.

17. The device of claim 1, wherein the channel member (82) defines a volume which is no greater than 300 nanolitres.

18. The device of claim 1, wherein the assay pad (20) comprises:
a first component (1102) constructed to separate red blood cells from plasma and further comprises a diffuse reflective material; a second component (1106) comprising the chemical reagent; and a third component (1104) comprising a polyamide-containing mesh.

19. The device of claim 18, wherein the diffuse reflective material comprises zirconium oxide.

20. The device of claim 18, wherein the second component comprises a membrane spin-coated with the chemical reagent.

21. The device of claim 18, wherein the second and third components are integrated by incorporating the chemical reagent into the polyamide-containing membrane.

## Patentansprüche

1. Vorrichtung zur Durchführung eines Assays, um die Konzentration eines Analyten in einer Körperflüssigkeitsprobe zu bestimmen, wobei die Vorrichtung umfasst:
ein Kanalelement (82) mit einem offenen Boden, einer ersten Öffnung und einer zweiten Öffnung, wobei die erste Öffnung an einem Ende des Kanalelements (82) zum Einbringen der Probe in das Kanalelement (82) und Ermöglichen eines Fließens der Probe dort hindurch ist;
ein Assay-Plättchen (20) in Kommunikation mit dem offenen Boden des Kanalelements (82) und das Kanalelement (82) abdeckend, wobei das Assay-Plättchen (20) ein Reagens umfasst, das geeignet ist, um eine chemische Reaktion auszulösen, wenn es dem Analyten ausgesetzt wird, wobei die chemische Reaktion eine Farbänderung in dem Assay-Plättchen (20) auslöst; und
eine lineare Anordnung (84) von optischen CMOS-Detektoren (85), die in Bezug auf das Assay-Plättchen (20) angeordnet ist, um die Farbänderung zu erkennen, wobei das Kanalelement (82) eine Länge definiert und die lineare Anordnung (84) von optischen CMOS-Detektoren mehrere CMOS-Sensoren aufweist, die eine Länge der Anordnung (84) definieren, die proportional zu der Länge des Kanalelements (82) ist.

2. Vorrichtung nach Anspruch 1, wobei der Analyt Glukose umfasst.

3. Vorrichtung nach Anspruch 1, wobei die Körperflüssigkeit Vollblut umfasst.

4. Vorrichtung nach Anspruch 1, wobei das Kanalelement (82) ein Mikrokanal ist.

5. Vorrichtung nach Anspruch 1, weiterhin umfassend eine Lichtquelle (22), die geeignet ist, um Licht bereitzustellen, das auf das Assay-Plättchen (20) einfällt.

6. Vorrichtung nach Anspruch 5, wobei die Lichtquelle (22) in Bezug auf das Kanalelement (82) und das Assay-Plättchen (20) eingerichtet ist, so dass Licht, das von der Lichtquelle übertragen wird, mindestens zum Teil durch das Kanalelement (82) und das Assay-Plättchen (20) übertragen wird, wobei die optischen Detektoren (85) derart eingerichtet sind, dass das übertragene Licht von den optischen Detektoren (85) empfangen wird.

7. Vorrichtung nach Anspruch 5, wobei die Lichtquelle (22) in Bezug auf das Kanalelement (82) und das Assay-Plättchen (20) eingerichtet ist, so dass Licht von der Lichtquelle übertragen wird und mindestens zum Teil von dem Assay-Plättchen (20) reflektiert wird, und die optischen Detektoren (85) in Bezug auf das Kanalelement (82) und das Assay-Plättchen (20) eingerichtet sind, um Licht zu empfangen, das von dem Assay-Plättchen (20) reflektiert wird.

8. Vorrichtung nach Anspruch 5, weiterhin umfassend einen Wellenleiter (32), der mit dem Assay-Plättchen (20) assoziiert ist, wobei die Lichtquelle (22) eingerichtet ist, so dass Licht, das davon abgestrahlt wird, auf jenen Wellenleiter (32) einfällt, wobei der Wellenleiter (32) in Bezug auf das Assay-Plättchen (20) eingerichtet ist, so dass Licht, das darin übertragen wird, von der Lichtquelle das Assay-Plättchen (20) beleuchtet, wobei das Assay-Plättchen (20) konstruiert ist, um das darauf einfallende Licht mindestens zum Teil zu reflektieren, und die optischen Detektoren (85) eingerichtet sind, um Licht zu empfangen, das von dem Assay-Plättchen (20) reflektiert wird.

9. Vorrichtung nach Anspruch 8, wobei der Wellenleiter (32) eingerichtet ist, um einen lateralen Rand des Assay-Plättchens (20) zu beleuchten, und die optischen Detektoren (85) eingerichtet sind, um reflektiertes Licht mittels des Wellenleiters (32) zu empfangen.

10. Vorrichtung nach Anspruch 8, wobei mindestens eine Seite des Wellenleiters (32) mit einer Antireflexbeschichtung versehen ist.

11. Vorrichtung nach Anspruch 1, weiterhin umfassend eine Linse (86), die zwischen den optischen Detektoren (85) und dem Assay-Plättchen (20) angeordnet ist.

12. Vorrichtung nach Anspruch 11, wobei die Linse (86) eine refraktive Linse ist.

13. Vorrichtung nach Anspruch 11, wobei die Linse (86) eine diffraktive Linse ist.

14. Vorrichtung nach Anspruch 11, wobei die Linse (86) integral mit den optischen Detektoren (85) ist.

15. Vorrichtung nach Anspruch 1, wobei die Vorrichtung weiterhin ein Hautpenetrationselement (12) zum Extrahieren der Körperflüssigkeitsprobe von einem Träger umfasst.

16. Vorrichtung nach Anspruch 1, wobei das Kanalelement (82) eine allgemeine Form eines länglichen, auf den Kopf gestellten U aufweist, wenn es in seiner betätigbaren Position innerhalb der Vorrichtung angeordnet ist.

17. Vorrichtung nach Anspruch 1, wobei das Kanalelement (82) ein Volumen definiert, das nicht größer als 300 Nanoliter ist.

18. Vorrichtung nach Anspruch 1, wobei das Assay-Plättchen (20) umfasst:
eine erste Komponente (1102), die konstruiert ist, um rote Blutkörperchen von Plasma zu trennen, und weiterhin ein diffus reflektierendes Material umfasst; eine zweite Komponente (1106), die das chemische Reagens umfasst; und eine dritte Komponente (1104), die ein polyamidhaltiges Maschengewebe umfasst.

19. Vorrichtung nach Anspruch 18, wobei das diffus reflektierende Material Zirkoniumoxid umfasst.

20. Vorrichtung nach Anspruch 18, wobei die zweite Komponente eine Membran umfasst, die mit dem chemischen Reagens rotationsbeschichtet ist.

21. Vorrichtung nach Anspruch 18, wobei die zweite und die dritte Komponente durch Einbinden des chemischen Reagens in die polyamidhaltige Membran integriert werden.

## Revendications

1. Dispositif de réalisation d'un dosage pour déterminer la concentration d'un analyte dans un échantillon de fluide corporel, le dispositif comprenant :
un élément de conduit (82) ayant un fond ouvert, un premier orifice et un deuxième orifice, dans lequel le premier orifice est à une extrémité de l'élément de conduit (82) pour l'introduction de l'échantillon dans l'élément de conduit (82), et permettant l'écoulement de l'échantillon à travers celui-ci ;
un tampon de dosage (20) en communication avec le fond ouvert de l'élément de conduit (82) et recouvrant l'élément de conduit (82), le tampon de dosage (20) comprenant un réactif adapté pour produire une réaction chimique lorsqu'il est exposé à l'analyte, la réaction chimique produisant un virage de couleur dans le tampon de dosage (20) ; et
un réseau linéaire (84) de détecteurs optiques CMOS (85) placés par rapport au tampon de dosage (20) de façon à détecter le virage de couleur, dans lequel l'élément de conduit (82) définit une longueur, et le réseau linéaire (84) de détecteurs optiques CMOS a une pluralité de capteurs CMOS qui définissent une longueur du réseau (84) qui est proportionnelle à la longueur de l'élément de conduit (82).

2. Dispositif selon la revendication 1, dans lequel l'analyte comprend du glucose.

3. Dispositif selon la revendication 1, dans lequel le fluide corporel comprend du sang total.

4. Dispositif selon la revendication 1, dans lequel l'élément de conduit (82) est un microconduit.

5. Dispositif selon la revendication 1, comprenant en outre une source de lumière (22) adaptée pour fournir de la lumière incidente sur le tampon de dosage (20).

6. Dispositif selon la revendication 5, dans lequel la source de lumière (22) est placée par rapport à l'élément de conduit (82) et au tampon de dosage (20) de telle façon que la lumière transmise depuis la source de lumière est transmise au moins en partie à travers l'élément de conduit (82) et le tampon de dosage (20), et les détecteurs optiques (85) sont placés de telle manière que la lumière transmise est reçue par les détecteurs optiques (85).

7. Dispositif selon la revendication 5, dans lequel la source de lumière (22) est placée par rapport à l'élément de conduit (82) et au tampon de dosage (20) de telle façon que la lumière est transmise depuis la source de lumière et réfléchie au moins en partie par le tampon de dosage (20), et les détecteurs optiques (85) sont placés par rapport à l'élément de conduit (82) et au tampon de dosage (20) pour recevoir la lumière réfléchie par le tampon de dosage (20).

8. Dispositif selon la revendication 5, comprenant en outre un guide d'ondes (32) associé au tampon de dosage (20), la source de lumière (22) est placée de telle façon que la lumière émise à partir de celle-ci est incidente sur ce guide d'ondes (32), le guide d'ondes (32) est placé par rapport au tampon de dosage (20) de telle façon que la lumière transmise dedans depuis la source de lumière éclaire le tampon de dosage (20), le tampon de dosage (20) est construit pour réfléchir au moins en partie la lumière incidente sur celui-ci, et les détecteurs optiques (85) sont placés pour recevoir la lumière réfléchie par le tampon de dosage (20).

9. Dispositif selon la revendication 8, dans lequel le guide d'ondes (32) est placé pour éclairer un bord latéral du tampon de dosage (20), et les détecteurs optiques (85) sont placés pour recevoir de la lumière réfléchie par le biais du guide d'ondes (32).

10. Dispositif selon la revendication 8, dans lequel au moins un côté du guide d'ondes (32) est pourvu d'un revêtement anti-réfléchissant.

11. Dispositif selon la revendication 1, comprenant en outre une lentille (86) disposée entre les détecteurs optiques (85) et le tampon de dosage (20).

12. Dispositif selon la revendication 11, dans lequel la lentille (86) est une lentille réfringente.

13. Dispositif selon la revendication 11, dans lequel la lentille (86) est une lentille diffractive.

14. Dispositif selon la revendication 11, dans lequel la lentille (86) est d'un seul tenant avec les détecteurs optiques (85).

15. Dispositif selon la revendication 1, dans lequel le dispositif comprend en outre un élément de pénétration cutanée (12) pour extraire l'échantillon de fluide corporel chez un porteur.

16. Dispositif selon la revendication 1, dans lequel l'élément de conduit (82) a généralement la forme d'un U inversé allongé lorsqu'il est disposé dans sa position opérationnelle à l'intérieur du dispositif.

17. Dispositif selon la revendication 1, dans lequel l'élément de conduit (82) définit un volume qui n'est pas supérieur à 300 nanolitres.

18. Dispositif selon la revendication 1, dans lequel le tampon de dosage (20) comprend :
un premier composant (1102) construit pour séparer les globules rouges du plasma et comprend en outre un matériau réfléchissant diffus ; un deuxième composant (1106) comprenant le réactif chimique ; et un troisième composant (1104) comprenant un maillage contenant du polyamide.

19. Dispositif selon la revendication 18, dans lequel le matériau réfléchissant diffus comprend de l'oxyde de zirconium.

20. Dispositif selon la revendication 18, dans lequel le deuxième composant comprend une membrane revêtue par centrifugation avec le réactif chimique.

21. Dispositif selon la revendication 18, dans lequel les deuxième et troisième composants sont intégrés en incorporant le réactif chimique dans la membrane contenant du polyamide.
